# EUROPEAN PATENT SPECIFICATION

(11) **EP 3 463 524 B1**
(45) Date of publication and mention of the grant of the patent: **20.01.2021**
(21) Application number: 17740475.3
(22) Date of filing: 05.06.2017
(51) Int. Cl.: A61M 5/315, A61M 5/24

(54) **SYRINGE FOR CARPULES**
SPRITZE FÜR KAPSELN
SERINGUE POUR DES CARPULES

(30) Priority: 06.06.2016 IT UA20164113
(43) Date of publication of application: 10.04.2019
(73) Proprietor: Pikdare-Società per Azioni, 22070 Casnate con Bernate CO (IT)
(72) Inventor: LEONARDI, Luca, 22070 Grandate CO (IT); POLLACI, Daniele, 22070 Grandate CO (IT); LURASCHI, Vittorio, 22070 Grandate CO (IT)
(74) Representative: Perani & Partners S.p.A.
(86) International application number: PCT/IB2017/053300
(87) International publication number: WO 2017/212388

(56) References cited:
- EP-A1- 1 967 161
- WO-A2-2007/005902
- DE-A1- 19 825 751
- FR-A- 1 186 571
- US-A1- 2013 023 885
- US-A1- 2015 045 740

## Description

The present invention relates to a syringe for carpules.

Carpule syringes are known, and are used for instance in dentistry. Such syringes have a body that extends mainly in a longitudinal direction and has a substantially cylindrical cavity. The body is designed to accommodate a carpule, i.e. a container for a fluid to be injected in the body of a patient. Particularly, carpules are generally provided with a sliding member and a discharging portion from which the fluid is ejected upon exertion of a pressure on the sliding member. Examples for syringes - not necessarily carpule syringes - can be found in EP 1 967 161, WO 2007/005902, US 2013/023885, US 2015/045740, DE 198 25 751 and FR 1 186 571.

The syringes have a plunger that, upon actuation by a user, slides relative to the body of the syringe and presses the carpule, and namely its sliding member, in the longitudinal direction, thereby causing the fluid contained therein to be ejected through a needle, which is supplied in combination with the syringe or separately therefrom.

Operating the syringe involves applying the needle to the syringe, introducing a carpule into the cavity in such a position that carpule discharge portion is located at the needle, then bringing the plunger at the sliding member of the carpule, and actuating the plunger to inject the fluid. These steps are manually carried out directly by the user of the syringe, who assembles the various parts.

For simpler operation, syringes should be desirably supplied to the user while having already carpules therein, by introducing the carpule during manufacture of the syringe or immediately after manufacture. For packaging reasons, the plunger should be desirably supplied in a condition in which it is removed from the syringe body.

The object of the present invention is to provide a carpule syringe solving the above discussed problem.

This and other objects are fulfilled by a carpule syringe as defined in one or more of the accompanying claims.

Advantageously, when the cover is in the open position, the carpule may be introduced into the cavity in a simple manner, which can be carried out by an industrial machinery.

Advantageously, once the carpule has been introduced and the cover has been moved to the closed position, the carpule is retained in the syringe body by the cover. Then, the syringe may be transported and distributed with the carpule therein, and not necessarily with the plunger inserted in the hole of the cover.

Further characteristics and advantages of the carpule syringe of the present invention will result from the following description of a preferred exemplary embodiment thereof, which is given by way of illustration and without limitation, with reference to the accompanying figures, in which:
- Figure 1 is an exploded perspective view of a first embodiment of a carpule syringe of the present invention,
- Figure 2 is sectional side view of the syringe of Figure 1 in a first operating configuration,
- Figure 3 is sectional side view of the syringe of Figure 1 in a second operating configuration,
- Figure 4 is a perspective view of a detail of the syringe of Figure 1.

Referring to the accompanying figures, numeral 1 generally designates a carpule syringe of the present invention, i.e. a syringe 1 for injecting a fluid initially contained in a carpule 2. The term carpule 2 is intended to designate a container, such as a vial, a cartridge or the like, generally made of glass and adapted to contain a dose of a medical fluid.

The syringe 1 comprises a body 3. The body 3 has a cavity 31 for receiving a fluid-containing carpule 2.

The carpule 2 extends between a first end 23 and a second end 24. The carpule 2 has a discharge opening 21 at the first end 23. The discharge portion 21 is configured for the outflowing carpule fluid to flow therethrough. Preferably, the carpule 2 comprises a protective membrane which engages the discharge opening 21 and is adapted to be perforated by a needle.

The carpule 2 comprises a sliding member 22, which is initially placed at the second end 24. The sliding member 22 is configured to slide in the carpule 2 from the second end 24 toward the first end 23, and press the fluid out of the carpule 2 through the discharge opening 21.

The body 1 has a first through opening 32 facing the cavity 31 and a second through opening 33 facing the cavity 31. In other words, the cavity 31 is a through cavity and opens out from the body 3 at the first opening 32 and at the second opening 33. In operation, the carpule 2 is disposed in the cavity 31 with the first end 23 facing the first opening 32 of the body 3, and the second end 24 facing the second opening 33 of the body 3.

Preferably, the body 3 extends mainly in a longitudinal direction X-X, and has a first end 34 and the second end 35 that are spaced apart in the longitudinal direction X-X. The first opening 32 and the second opening 33 of the body 3 are located at the first end 34 and the second end 35 of the body 3 respectively.

The first opening 32 is adapted to be engaged by a needle 4 which may be part of the syringe 1 or, preferably, may be supplied separately from the syringe 1. The syringe 1 may further comprise a connecting member 7 which is configured to secure the needle 4 to the body 3.

Particularly, the connecting member 7 has a hole 721 for the needle 4 to extend therethrough. The connecting member 7 has threads on an inner surface. The body 3 has a neck 36 located substantially at the first opening 32. The neck 36 has threads on an outer surface thereof. The connecting member 7 is adapted to be tightened on the neck 36, particularly by mating engagement on the outer threads of the neck 36 and the inner thread of the connecting member 7. The needle 4 is configured to perforate the membrane that closes the discharge opening 21 of the carpule 2, as the connecting member 7 is tightened on the neck 36.

The syringe 1 may further comprise a removable protective cap 8, which is configured to cover the needle 4. The cap 8 is configured to be secured to the neck 36 or to the connecting member 7.

The second opening 33 of the body 3 is configured to allow the carpule 2 to be introduced into the cavity 31 of the body 3.

Preferably, the body 3 of the syringe 1 is made of a plastic material. Advantageously, the body 3 may have a low cost and a light weight, as particularly desired in the manufacture of a disposable syringe 1. A disposable syringe 1 advantageously does not require sterilization after use.

The syringe 1 comprises a plunger 6 configured to evacuate the fluid from the carpule 2. The plunger 6 is adapted to be actuated to push the sliding member 22 of the carpule 2. Particularly, the plunger 6 is adapted to be inserted into the second opening 33. It shall be noted that the plunger 6 may be supplied in a condition in which it has been removed from the second opening 33 and is adapted to be easily introduced therein by a user, which constitutes an advantage for packaging of the syringe 1. Further details of the plunger 6 will be provided later herein.

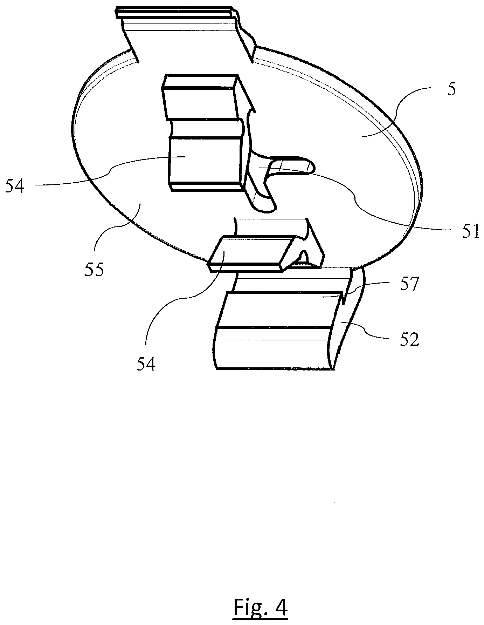

In one aspect of the invention, the syringe 1 comprises a cover 5 as shown, for instance, in Figure 4. The cover 5 is attached to the body 3 of the syringe 1 and has the purpose of closing the aforementioned second opening 33. In the illustrated embodiment, the cover 5 is pivotally connected to the body 3 of the syringe 1 by means of a hinge. Preferably, the cover 3 is made of a plastic material, which provides the same advantages as described when the body 3 is made of plastic.

Particularly, the cover 5 is adapted to be switched between a closed position, in which the cover 5 engages the second opening 33, and an open position, in which the second opening 33 is free. The cover 5 has an inner surface 55 and an outer surface 56, opposite to the inner surface 55. The inner surface 55 faces the cavity 31 when the cover 5 is in the closed position.

When the cover 5 is in the open position the carpule 2 can be introduced into and/or removed out of the cavity 31 through the second opening 33.

When the cover 5 is in the closed position the carpule 2 cannot be introduced into and/or removed out of the cavity 31 through the second opening 33. Advantageously, when the carpule 2 is within the cavity 31 of the body 3 and the cover 5 is in the closed position, there is no risk that the carpule 2 may accidentally come out of the syringe 1.

The cover 5 has a hole 51 for receiving the aforementioned plunger 6. When the cover 5 is in the closed position, the plunger 6 may be slidingly introduced into the hole 51 of the cover 5, particularly through the second opening 433 of the body 3.

Preferably, the cover 5 comprises a locking member 52. In the embodiment of the annexed figures, the locking member 52 is defined by a protuberance that projects from an edge of the cover 5. The protuberance of the locking member 52 is situated in a position of the cover 5 that is distal from the hinge that connects the cover 5 to the body 3 of the syringe 1.

The body 3 of the syringe 1 has a seat 37 formed therein. The protuberance of the locking member 52 is configured to irreversibly grip into the seat 37 and maintain the cover 5 in the closed position.

The seat 37 is located substantially at the second end 35 of the body 3 of the syringe 1, proximate to the second opening 33. The seat 37 is particularly formed outside the cavity 31. The seat 37 is also shaped complementary to the protuberance of the locking member 52, thereby holding it therein irreversibly.

In the closed position, the protuberance is located outside the cavity 31. Particularly, when the cover 5 moves from the open position to the closed position, the protuberance elastically deforms and grips into the seat 37 by a snap engagement. During such snap engagement, the protuberance 52 bends toward the seat 37, i.e. toward the body 3 of the syringe 1.

The locking member 52 has an inner surface and an outer surface opposite thereto. The inner surface of the locking member 52 faces the body 3 of the syringe 1 when the cover 5 is in the closed position. Therefore, the inner surface of the locking member 52 is oriented also toward the hinge. The protuberance has a holding pawl 57 which projects from the inner surface of the locking member 52, and which hence is oriented substantially toward the hinge and/or toward the body 3 of the syringe 1 too.

The seat 37 defines a step 38, which in the closed position is opposed to the holding pawl 57, the step 38 facing and abutting the holding pawl 57 to prevent the cover 5 from moving from the closed position to the open position. In other words, the step 38 is formed to prevent the holding pawl 57 from sliding in the longitudinal direction X-X away from the body 3 of the syringe 1, and to prevent the cover 5 to pivot relative to the hinge. More in detail, the step 38 is disposed between the holding pawl 57 and the second end 35 of the body 3 of the syringe 1.

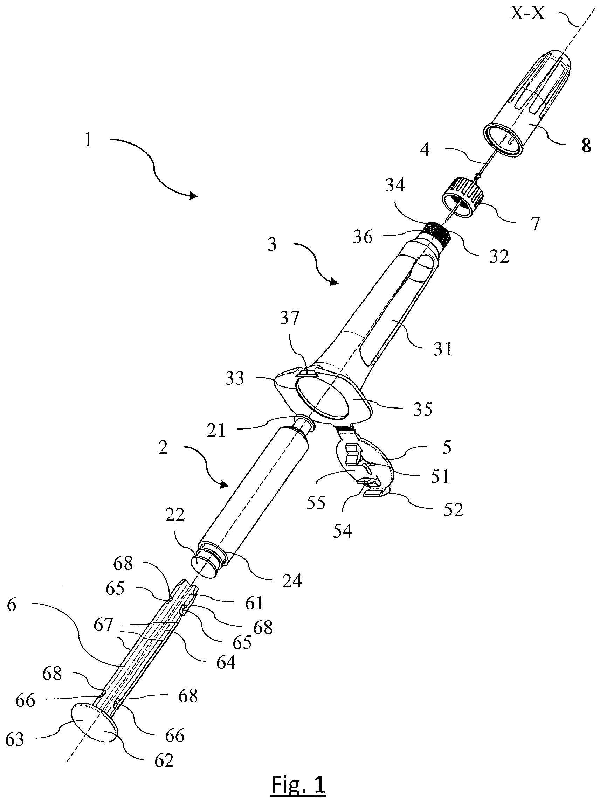

When making a disposable syringe 1, as particularly shown in Figures 1 to 3, the protuberance of the locking member 52 is configured to irreversibly grip into the seat 35, i.e. the locking member 52 has no unlocking means. Particularly, in the closed position the outer surface of the locking member 52 has no projections adapted to be seized by a user, and is preferably smooth.

Advantageously, when the locking member 52 grips into the seat 37, the cover 5 is prevented from moving from the closed position to the open position, and the carpule 2 is prevented from coming out of the cavity 31 of the body 3 of the syringe 1. In fact, bringing the cover 3 to the open position would require first taking the holding pawl 57 away from abutment with the step 38 of the seat. This involves elastically deforming the protuberance away from the seat 37 and from the body 3 of the syringe 1. The lack of gripping projections on the outer surface of the locking member 52 leaves almost no chance of manually obtaining such deformation.

The plunger 6 has a first end portion 61 and a second end portion 62 opposite to the first end portion 61. The first end portion 61 is configured to be introduced into the cavity 31 of the body 3 through the second opening 33, particularly through the hole 51 of the cover 5 when the cover 5 is in the closed position.

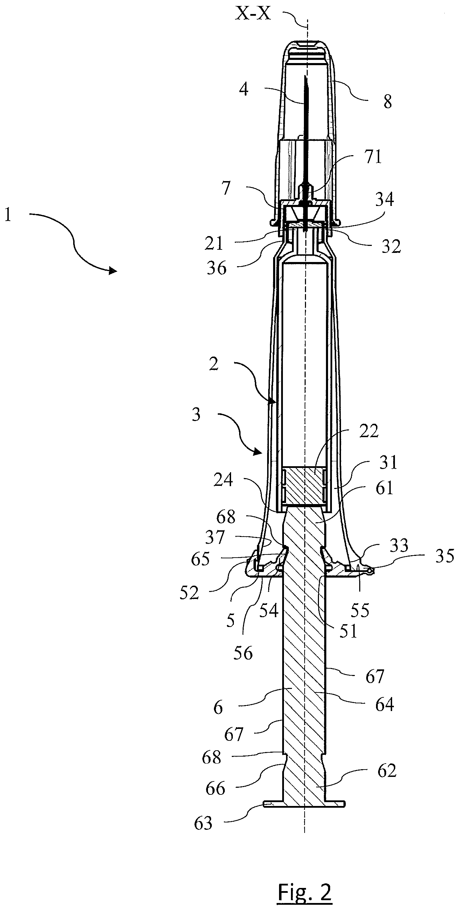

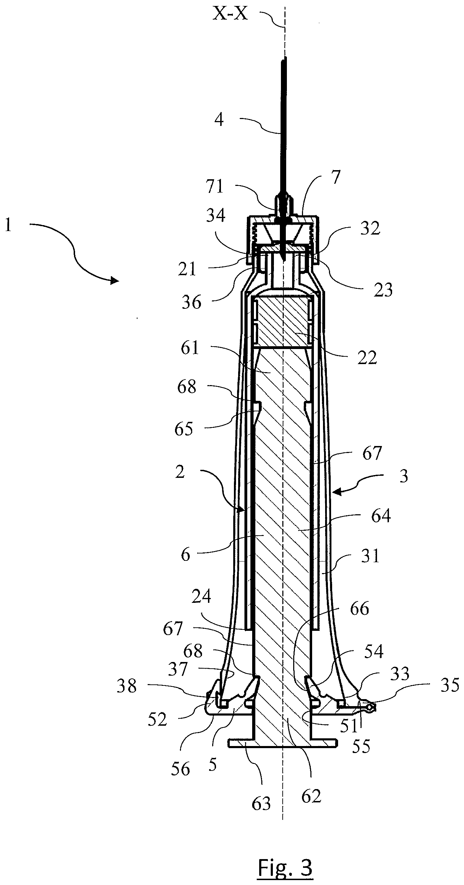

In detail, the plunger 6 may be placed in start limit position, as shown for instance in Figure 2, and in an end limit position as shown in Figure 3. In the start limit position only the first end portion 61 is inserted in the cavity 31. In the end limit position only the second end portion 62 is inserted in the cavity 31. The plunger 6 may slide in the longitudinal direction X-X through the hole 51 of the cover 5 from the start limit position to the end limit position.

The plunger 6 has a head 63, at the second end portion 62, and a rod 64. The rod 64 extends between the first and second end portions 61, 62. Furthermore, the rod 64 has four flaps 67. Each flap 67 extends between the head 63 and the first end portion 61 of the plunger 6. The flaps 67 are in a cross arrangement.

The hole 51 of the cover 5 is configured to prevent the head 63 of the plunger 62 from sliding through the hole 51. In other words, the cover 5 is configured to prevent the plunger 6 from being fully introduced into the cavity 31 of the body 3 of the syringe 1. In other words, the hole 51 have the same size as the rod 64 but is smaller than the head 63. The hole 51 had a cross shape, to allow the flaps 67 of the rod 6 to slide in the longitudinal direction X-X.

In the embodiment as shown in Figures 1 to 3, the syringe 1 comprises retention means 54, 65, 66, which are configured to lock the plunger 6 in the start limit position and/or in the end limit position.

Preferably the retention means 54, 65, 66 are configured to prevent the plunger 6 from sliding from the end limit position to the start limit position. Furthermore, the retention means 54, 65, 66 are configured to allow the plunger 6 to slide from the start limit position to the end limit position.

Advantageously, as the plunger 6 is locked in the start limit position, a user may be safely prevented from causing leakage of part of the fluid of the carpule 2, as he/she introduces the plunger 6 into the hole 51 of the cover 5, before starting the injection.

Advantageously, as the plunger 6 is prevented from sliding from the end limit position to the start limit position, the syringe 1 may be safely prevented from being reused after first use. Particularly, there is no risk that the user may use the syringe 1 on a patient and then reuse it, for instance on a different patient, and inadvertently forget to sterilize the syringe 1.

Preferably, the retention means 54, 65, 66 comprise at least one notch 65, 66 located on the plunger 6, and at least one retention member 54 associated with the cover 5. The retention member 54 is configured to engage in the notch 65, 66 and prevent removal of the plunger 6 from the hole 51 of the cover 5. Particularly, the retention member is configured for elastic deformation and snappy engagement in the notch 65, 66. The retention member 54 projects from the inner surface 55 of the cover 5, and particularly faces the interior of the cavity 31 when the cover 5 is in the closed position.

Preferably, the retention means 54, 65, 66 comprise a first notch 65 at the first end position 61 of the plunger 6 and a second notch 66 at the second end portion 62 of the plunger 6. Particularly, the first and second notches 65, 66 are formed on the rod 64 of the plunger 6. The first and second notches 65, 66 are recessed with respect to the rod 64 in a direction transverse to the longitudinal direction X-X, when the rod 64 is inserted in the hole 51 of the cover 5 in the closed position.

The retention member 54 is configured to engage in the first notch 65 to lock the plunger 6 in the start limit position. The retention member 54 is also configured to engage in the second notch 66 to lock the plunger 6 in the end limit position.

The first notch 65 and the second notch to 66 may be a plurality of first notches 65 and a plurality of second notches 66 respectively. The plurality of first notches 65 are all located in one first longitudinal position of the rod 64, and the plurality of second notches 66 are all located in one second longitudinal position of the rod 64. Each of the first notches 65 and each of the second notches 66 are located on corresponding flaps 67 of the rod 6.

Furthermore, the retention means 54, 65, 66 comprise a pair or a plurality of retention members 54. Particularly, each retention member 54 is configured to engage in the first and/or the second notches 65, 66 corresponding to a respective flap 67.

Preferably, each notch 65, 66 is inclined to the longitudinal direction X-X, to allow the passage of the retention member 54 as the plunger 6 is introduced into the cavity 31, and to prevent the passage of the retention member 54 in the opposite direction, thereby preventing the plunger 6 from being removed from the cavity 31.

Particularly, the plurality of first notches 65 and the plurality of second notches 66 are inclined to the longitudinal direction X-X to converge towards the first end portion 61 of the plunger 6.

Furthermore, each notch 65, 66 has a right-angled corner 68, which is configured to oppose the respective retention member 54, as the plunger 6 is removed from the cavity 31.

## Claims

1. A syringe (1) for carpules (2), comprising:
- a body (3) having a cavity (31) for receiving a fluid-containing carpule (2), said body (3) having a first through opening (32), facing the cavity (31) and adapted to be engaged by a needle (4), and a second through opening (33), facing the cavity (31) and configured to allow insertion of said carpule (2) into said cavity (31);
- a plunger (6) configured to evacuate said fluid from the carpule (2);
- a cover (5) attached to said body (3) and adapted to be switched between a closed position, in which the cover (5) engages said second opening (33), and an open position, in which said second opening (33) is free,
- said cover (5) having a hole (51), said plunger (6) being configured to be slidingly inserted in said hole (51), when said cover (5) is in said closed position,
**characterized in that:**
- said cover (5) comprises a locking member (52), which is defined by a protuberance projecting from an edge of the cover (5),
- said body (3) has a seat (37), said protuberance being configured to irreversibly grip into said seat (37) and maintain the cover (5) in the closed position, the seat (37) being shaped complementarily to the protuberance, thereby holding it therein irreversibly.

2. A syringe (1) as claimed in claim 1, wherein said body (3) and/or said cover (5) are made of plastic.

3. A syringe (1) as claimed in claim 1 or 2, wherein:
- the seat (37) is formed outside the cavity (31), and
- in the closed position, the protuberance is located outside the cavity (31).

4. A syringe (1) as claimed in claim 3, wherein, during the transition from the open position to the closed position, the protuberance of the locking member (52) is designed to elastically deform and grip into the seat (37) by snap engagement, and to bend toward the body (3) of the syringe (1) during such snap engagement.

5. A syringe (1) as claimed in any of claims 1 to 4, wherein the cover (5) is pivotally connected to the body (3) of the syringe (1) by means of a hinge.

6. A syringe (1) as claimed in claim 5, wherein the protuberance is placed in a position of the cover (5) which is distal from the hinge.

7. A syringe (1) as claimed in any of claims 1 to 6, wherein:
- the locking member (52) has an inner surface, which faces the body (3) of the syringe (1) when the cover (5) is in the closed position, and an outer surface opposite to the inner surface,
- the outer surface of the locking member (52) has no projections adapted to be be seized by a user.

8. A syringe (1) as claimed in claim 7, wherein the outer surface of the locking member (52) is smooth.

9. A syringe (1) as claimed in claim 7 or 8, wherein:
- the protuberance has a holding pawl (57) projecting from the inner surface of the locking member (52), and
- the seat (37) defines a step (38), which in the closed position faces and abuts the holding pawl (57) to prevent the cover (5) from moving from the closed position to the open position.

10. A syringe (1) as claimed in any of the preceding claims, comprising retention means (54, 65, 66) configured to lock said plunger (6) in a start limit position and/or an end limit position, wherein the retention means (54, 65, 66) comprise:
- at least one notch (65, 66) on said plunger (6), and
- at least one retention member (54) associated with said cover (5) and configured to engage in said notch (65, 66) and prevent removal of the plunger (6).

11. A syringe (1) as claimed in claim 10, wherein said retention means (54, 65, 66) are configured to prevent said plunger (6) from sliding from said end limit position to said start limit position.

12. A syringe (1) as claimed in claim 10 or 11, wherein:
- said plunger (6) has a first end portion (61) and a second end portion (62) opposite to said first end portion (61), said first end portion (61) being configured to be introduced into the cavity (31) through the second opening (33),
- said retention means (54, 65, 66) comprising a first notch (65) at said first end portion (61), said retention member (54) being configured to engage in said first notch (65) to lock said plunger (6) in said start limit position;
- said retention means (54, 65, 66) comprising a second notch (66) at said second end portion (62), said retention member (54) being configured to engage in said second notch (65) to lock said plunger (6) in said end limit position.

13. A kit comprising a syringe (1) as claimed in any of the preceding claims and a carpule (2) inserted in the cavity (31) of the body (3) of said syringe (1).

## Patentansprüche

1. Spritze (1) für Karpulen (2), die Folgendes umfasst:
- einen Körper (3) mit einem Hohlraum (31) zum Aufnehmen einer Fluid enthaltenden Karpule (2), wobei der Körper (3) eine erste Durchgangsöffnung (32), die dem Hohlraum (31) zugewandt und an den Eingriff einer Nadel (4) angepasst ist, und eine zweite Durchgangsöffnung (33), die dem Hohlraum (31) zugewandt und dazu ausgelegt ist, das Einstecken der Karpule (2) in den Hohlraum (31) zu erlauben;
- einen Kolben (6), der dazu ausgelegt ist, das Fluid aus der Karpule (2) zu entfernen;
- eine Abdeckung (5), die am Körper (3) befestigt und angepasst ist, zwischen einer geschlossenen Position, in der die Abdeckung (5) in die zweite Öffnung (33) eingreift, und einer offenen Position, in der die zweite Öffnung (33) frei ist, umgesetzt zu werden,
- wobei die Abdeckung (5) ein Loch (51) aufweist, wobei der Kolben (6) dazu ausgelegt ist, gleitend in das Loch (51) eingesteckt zu werden, wenn sich die Abdeckung (5) in der geschlossenen Position befindet,
**dadurch gekennzeichnet, dass:**
- die Abdeckung (5) ein Verriegelungselement (52) umfasst, das durch einen Vorsprung, der von einem Rand der Abdeckung (5) vorsteht, definiert ist,
- der Körper (3) einen Sitz (37) aufweist, wobei der Vorsprung dazu ausgelegt ist, irreversibel in den Sitz (37) zu greifen und die Abdeckung (5) in der geschlossenen Position zu halten, wobei der Sitz (37) komplementär zum Vorsprung geformt ist, wodurch er irreversibel darin gehalten wird.

2. Spritze (1) nach Anspruch 1, wobei der Körper (3) und/oder die Abdeckung (5) aus Kunststoff bestehen.

3. Spritze (1) nach Anspruch 1 oder 2, wobei:
- der Sitz (37) außerhalb des Hohlraums (31) gebildet ist und
- der Vorsprung in der geschlossenen Position sich außerhalb des Hohlraums (31) befindet.

4. Spritze (1) nach Anspruch 3, wobei der Vorsprung des Verriegelungselements (52) konzipiert ist, sich während des Übergangs von der offenen Position zur geschlossenen Position elastisch zu verformen und durch Rasteingriff in den Sitz (37) zu greifen und sich während des Rasteingriffs zum Körper (3) der Spritze (1) hin zu biegen.

5. Spritze (1) nach einem der Ansprüche 1 bis 4, wobei die Abdeckung (5) mittels eines Scharniers schwenkbar mit dem Körper (3) der Spritze (1) verbunden ist.

6. Spritze (1) nach Anspruch 5, wobei der Vorsprung in einer Position der Abdeckung (5) platziert ist, die distal vom Scharnier ist.

7. Spritze (1) nach einem der Ansprüche 1 bis 6, wobei:
- das Verriegelungselement (52) eine Innenfläche, die dem Körper (3) der Spritze (1) zugewandt ist, wenn sich die Abdeckung (5) in der geschlossenen Position befindet, sowie eine Außenfläche gegenüber der Innenfläche aufweist,
- die Außenfläche des Verriegelungselements (52) keine Projektionen aufweist, die angepasst sind, von einem Benutzer ergriffen zu werden.

8. Spritze (1) nach Anspruch 7, wobei die Außenfläche des Verriegelungselements (52) glatt ist.

9. Spritze (1) nach Anspruch 7 oder 8, wobei:
- der Vorsprung eine Halteklinke (57) aufweist, die von der Innenfläche des Verriegelungselements (52) vorsteht, und
- der Sitz (37) eine Stufe (38) definiert, die in der geschlossenen Position der Halteklinke (57) zugewandt ist und an derselben anliegt, um zu verhindern, dass sich die Abdeckung (5) aus der geschlossenen Position in die offene Position bewegt.

10. Spritze (1) nach einem der vorhergehenden Ansprüche, die Rückhaltemittel (54, 65, 66) umfasst, die dazu ausgelegt sind, den Kolben (6) in einer Anfangsbegrenzungsposition und/oder einer Endbegrenzungsposition zu verriegeln, wobei die Rückhaltemittel (54, 65, 66) Folgendes umfassen:
- mindestens eine Kerbe (65, 66) am Kolben (6) und
- mindestens ein Rückhalteelement (54), das mit der Abdeckung (5) verknüpft und dazu ausgelegt ist, in die Kerbe (65, 66) einzugreifen und die Entfernung des Kolbens (6) zu verhindern.

11. Spritze (1) nach Anspruch 10, wobei die Rückhaltemittel (54, 65, 66) dazu ausgelegt sind zu verhindern, dass der Kolben (6) von der Endbegrenzungsposition zur Anfangsbegrenzungsposition gleitet.

12. Spritze (1) nach Anspruch 10 oder 11, wobei:
- der Kolben (6) einen ersten Endabschnitt (61) und einen zweiten Endabschnitt (62) gegenüber dem ersten Endabschnitt (61) aufweist, wobei der erste Endabschnitt (61) dazu ausgelegt ist, durch die zweite Öffnung (33) in den Hohlraum (31) eingeführt zu werden,
- die Rückhaltemittel (54, 65, 66) eine erste Kerbe (65) im ersten Endabschnitt (61) umfassen, wobei das Rückhalteelement (54) dazu ausgelegt ist, in die erste Kerbe (65) einzugreifen, um den Kolben (6) in der Anfangsbegrenzungsposition zu verriegeln;
- die Rückhaltemittel (54, 65, 66) eine zweite Kerbe (66) im zweiten Endabschnitt (62) umfassen, wobei das Rückhalteelement (54) dazu ausgelegt ist, in die zweite Kerbe (65) einzugreifen, um den Kolben (6) in der Endbegrenzungsposition zu verriegeln.

13. Kit, das eine Spritze (1) nach einem der vorhergehenden Ansprüche und eine Karpule (2), die in den Hohlraum (31) des Körpers (3) der Spritze (1) eingesteckt ist, umfasst.

## Revendications

1. Seringue (1) pour des carpules (2), comprenant :
- un corps (3) ayant une cavité (31) pour recevoir une carpule (2) contenant un fluide, ledit corps (3) ayant une première ouverture traversante (32), faisant face à la cavité (31) et adaptée pour être engagée par une aiguille (4), et une seconde ouverture traversante (33), faisant face à la cavité (31) et configurée pour permettre l'insertion de ladite carpule (2) dans ladite cavité (31) ;
- un piston (6) configuré pour évacuer ledit fluide de la carpule (2) ;
- un couvercle (5) fixé audit corps (3) et adapté pour être commuté entre une position fermée, où le couvercle (5) s'engage dans ladite seconde ouverture (33), et une position ouverte, où ladite seconde ouverture (33) est libre,
- ledit couvercle (5) ayant un trou (51), ledit piston (6) étant configuré pour être inséré de manière coulissante dans ledit trou (51), lorsque ledit couvercle (5) est dans ladite position fermée,
**caractérisé en ce que** :
- ledit couvercle (5) comprend un élément de verrouillage (52), qui est défini par une protubérance faisant saillie depuis un bord du couvercle (5),
- ledit corps (3) comporte un siège (37), ladite protubérance étant configurée pour s'agripper de manière irréversible dans ledit siège (37) et maintenir le couvercle (5) en position fermée, le siège (37) étant formé de manière complémentaire à la protubérance, le maintenant ainsi de manière irréversible dans celui-ci.

2. Seringue (1) selon la revendication 1, où ledit corps (3) et/ou ledit couvercle (5) sont en matière plastique.

3. Seringue (1) selon la revendication 1 ou 2, où :
- le siège (37) est formé à l'extérieur de la cavité (31), et
- en position fermée, la protubérance est située à l'extérieur de la cavité (31).

4. Seringue (1) selon la revendication 3, où, pendant la transition de la position ouverte à la position fermée, la protubérance de l'élément de verrouillage (52) est conçue pour se déformer élastiquement et s'agripper dans le siège (37) par encliquetage, et pour se courber vers le corps (3) de la seringue (1) pendant un tel encliquetage.

5. Seringue (1) selon l'une quelconque des revendications 1 à 4, où le couvercle (5) est relié de manière pivotante au corps (3) de la seringue (1) au moyen d'une charnière.

6. Seringue (1) selon la revendication 5, où la protubérance est placée dans une position du couvercle (5) qui est distale par rapport à la charnière.

7. Seringue (1) selon l'une quelconque des revendications 1 à 6, où :
- l'élément de verrouillage (52) comporte une surface intérieure, qui fait face au corps (3) de la seringue (1) lorsque le couvercle (5) est en position fermée, et une surface extérieure opposée à la surface intérieure,
- la surface extérieure de l'élément de verrouillage (52) ne comporte pas de saillies conçues pour être saisies par un utilisateur.

8. Seringue (1) selon la revendication 7, où la surface extérieure de l'élément de verrouillage (52) est lisse.

9. Seringue (1) selon la revendication 7 ou 8, où :
- la protubérance comporte un cliquet de retenue (57) faisant saillie à partir de la surface intérieure de l'élément de verrouillage (52), et
- le siège (37) définit un cran (38) qui, en position fermée, fait face au cliquet de retenue (57) et vient en butée contre celui-ci pour empêcher le couvercle (5) de passer de la position fermée à la position ouverte.

10. Seringue (1) selon l'une quelconque des revendications précédentes, comprenant des moyens de retenue (54, 65, 66) configurés pour verrouiller ledit piston (6) dans une position de début de course et/ou une position de fin de course, où les moyens de retenue (54, 65, 66) comprennent :
- au moins une encoche (65, 66) sur ledit piston (6), et
- au moins un élément de retenue (54) associé audit couvercle (5) et configuré pour s'engager dans ladite encoche (65, 66) et empêcher le retrait du piston (6).

11. Seringue (1) selon la revendication 10, où lesdits moyens de retenue (54, 65, 66) sont configurés pour empêcher ledit piston (6) de coulisser de ladite position de fin de course à ladite position de début de course.

12. Seringue (1) selon la revendication 10 ou 11, où :
- ledit piston (6) comporte une première partie d'extrémité (61) et une seconde partie d'extrémité (62) opposée à ladite première partie d'extrémité (61), ladite première partie d'extrémité (61) étant configurée pour être introduite dans la cavité (31) par la seconde ouverture (33),
- lesdits moyens de retenue (54, 65, 66) comprenant une première encoche (65) au niveau de ladite première partie d'extrémité (61), ledit élément de retenue (54) étant configuré pour s'engager dans ladite première encoche (65) pour verrouiller ledit piston (6) dans ladite position de départ de course ;
- lesdits moyens de retenue (54, 65, 66) comprenant une seconde encoche (66) au niveau de ladite seconde partie d'extrémité (62), ledit élément de retenue (54) étant configuré pour s'engager dans ladite seconde encoche (65) pour verrouiller ledit piston (6) dans ladite position de fin de course.

13. Kit comprenant une seringue (1) selon l'une quelconque des revendications précédentes et une carpule (2) insérée dans la cavité (31) du corps (3) de ladite seringue (1).
